# EUROPEAN PATENT SPECIFICATION

(11) **EP 1 848 425 B1**
(45) Date of publication and mention of the grant of the patent: **17.08.2011**
(21) Application number: 06706679.5
(22) Date of filing: 06.02.2006
(51) Int. Cl.: A61K 31/40, A61K 31/496, A61P 9/10

(54) **THERAPEUTIC COMBINATIONS OF MANIDI PINE AND SIMVASTATIN**
THERAPEUTISCHE KOMBINATIONEN AUS MANIDIPIN UND SIMVASTATIN
COMBINAISONS THERAPEUTIQUES DE MANIDIPINE ET DE SIMVASTATINE

(30) Priority: 17.02.2005 EP 05003382
(43) Date of publication of application: 31.10.2007
(73) Proprietor: CHIESI FARMACEUTICI S.p.A., 43100 Parma (IT)
(72) Inventor: FOGARI, Roberto, Chiesi Farmaceutici S.p.A., I-43100 Parma (IT); DELL'ANNA, Carmen, Chiesi Farmaceutici S.p.A., I-43100 Parma (IT)
(74) Representative: Minoja, Fabrizio
(86) International application number: PCT/EP2006/001024
(87) International publication number: WO 2006/087117

(56) References cited:
- WO-A-99/11260
- FOGARI ROBERTO ET AL: "Effect of amlodipine-atorvastatin combination on fibrinolysis in hypertensive hypercholesterolemic patients with insulin resistance" AMERICAN JOURNAL OF HYPERTENSION, vol. 17, no. 9, September 2004 (2004-09), pages 823-827, XP002376725 ISSN: 0895-7061
- ZANCHETTI ALBERTO ET AL: "Efficacy, tolerability, and impact on quality of life of long-term treatment with manidipine or amlodipine in patients with essential hypertension" JOURNAL OF CARDIOVASCULAR PHARMACOLOGY, vol. 38, no. 4, October 2001 (2001-10), pages 642-650, XP009065066 ISSN: 0160-2446
- JUKEMA J WOUTER ET AL: "Proposed synergistic effect of calcium channel blockers with lipid-lowering therapy in retarding progression of coronary atherosclerosis" CARDIOVASCULAR DRUGS AND THERAPY, vol. 12, no. SUPPL. 1, April 1998 (1998-04), pages 111-118, XP002376726 ISSN: 0920-3206
- JUKEMA J WOUTER ET AL: "Amlodipine and atorvastatin in atherosclerosis: a review of the potential of combination therapy." EXPERT OPINION ON PHARMACOTHERAPY. FEB 2004, vol. 5, no. 2, February 2004 (2004-02), pages 459-468, XP009065081 ISSN: 1465-6566
- LEFER ALLAN M ET AL: "Simvastatin preserves the ischemic-reperfused myocardium in normocholesterolemic rat hearts", CIRCULATION, vol. 100, no. 2, 13 July 1999 (1999-07-13) , pages 178-184, ISSN: 0009-7322

## Description

The invention relates to (±)-1,4-dihydro-2,6-dimethyl-4-(3-nitrophenyl)-3,5-pyridinedicarboxylic acid 2-[4-(diphenylmethyl)-1-piperazinyl]ethyl methyl ester (manidipine) or a physiologically acceptable salt thereof in combination with simvastatin or a physiologically acceptable salt thereof to protect the myocardium from the damages associated to post-ischemic ventricular dysfunction at reperfusion.

In a particular embodiment, the invention relates to a medicament comprising a fixed combination of manidipine or a physiologically acceptable salt thereof with simvastatin or a physiologically acceptable salt thereof.

In a further embodiment, the invention also relates to a pharmaceutical composition for oral administration comprising said fixed combination, and to a kit comprising: a) a therapeutically effective amount of manidipine or a physiologically acceptable salt thereof and a pharmaceutically acceptable carrier or diluent in a first unit dosage form; b) a therapeutically effective amount of simvastatin or a physiologically acceptable salt thereof and a pharmaceutically acceptable carrier or diluent in a second unit dosage form; and c) container means for containing said first and second dosage forms.

### BACKGROUND OF THE INVENTION

Cardiovascular diseases, disorders or events, affecting the heart and/or associated blood vessels, are the main cause of morbidity and one of the most common cause of death.

They are mostly caused by atherosclerosis which is a series of modifications constituted by thickening and loss of elasticity of the wall of an arterial vessel.

Hypertension and hyperlipidemia, in particular hypercholesterolemia, have been considered the major risk factors for the development of atherosclerosis.

In the last years newer risk factors for the development of atherosclerosis have been identified and include inflammation and its markers such as C-reactive protein (CRP), oxidative stress and endothelial dysfunction.

More recently, clinical, pathological and experimental observations strongly support a role for systemic inflammation in the pathogenesis of a cardiovascular disease (Jain M et al Nat Rev Drug Discov 2005, 4(12), 977-987).

In endothelial dysfunction, the homeostasis of vasoactive substances is disrupted. The level of the vasoconstrictor factor angiotensin II, promoting the proliferation and influx of vascular cell adhesion molecule-1 (VCAM-1), intercellular adhesion molecule-1 (ICAM-1) and cytokines and the level of the vasoconstrictor hormone endothelin-1 (ET-1) are increased.

In hypertension, a close relationship has also been demonstrated between disorders of lipid metabolism and impaired fibrinolysis, mainly expressed as increased plasminogen activator inhibitor type 1 (PAI-1) levels and depressed tissue plasminogen activator (t-PA) activity.

Current hypertension treatment guidelines stress the role of total risk factor management and state not only to lower blood pressure but also to lower the levels of low-density lipoproteins (LDL) cholesterol to improve the global cardiovascular risk profile.

Drug therapy hypertension is currently based on the use of different drug classes, including angiotensin-converting enzyme (ACE) inhibitors, angiotensin receptor blockers (ARBs), diuretics, ß-blockers, and calcium-channel blockers -CCBs- (also referred to as calcium antagonists), that can be separated into two main groups, i.e. the dihydropyridine and the non-dihydropyridine derivatives.

Some dihydropyridine calcium-channel blockers seem to possess antiatherosclerotic properties.

Manidipine, (±)-1,4-dihydro-2,6-dimethyl-4-(3-nitrophenyl)-3,5-pyridinedicarboxylic acid 2-[4-(diphenylmethyl)-1-piperazinyl]ethyl methyl ester is a long acting calcium-channel blocker belonging to dihydropyridine class. It was first disclosed in the European patent EP 94195.

Hypercholesterolemia in turn is currently managed with the 3-hydroxy-3-methylglutaryl coenzyme A (HMG-CoA) reductase inhibitors, commonly referred to as statins.

Statins currently available on the market are lovastatin, simvastatin, pravastatin, fluvastatin, atorvastatin, pitavastatin and rosuvastatin.

It is well known that statins lower the levels of LDL-cholesterol.

Moreover, among the several ancillary effects of statins, it has been reported in the literature that they also possess anti-inflammatory properties and it has been suggested that their atheroprotective effects can be due not only to the lipid-lowering effect but also to their anti-inflammatory properties. In particular some statins improve endothelial function as reflected by the reduction of the ICAM-1 and IL-6 levels (Nawawi H et al Atherosclerosis 2003, 169, 283-291).

Recently, it has been reported (Ridker P M et al New Engl J Med 2005, 352, 20-28) that statins lower CRP levels in a manner largely independent of LDL cholesterol levels and that the level of CRP achieved as a result of statin therapy may have clinical relevance analogous to that of the LDL cholesterol levels achieved with the same therapy.

On the contrary, contrasting data have been reported about the effects of dihydropyridine CCBs on the CRP levels.

Yasunari K et al J Am Coll Cardiol 2004, 43, 2116-2123) found that amlodipine does not significantly modify the CRP levels in hypertensive patients, whereas Takase H et al (Am J Cardiol, May 2005, 95, 1235-1237 issued on-line on 9.07.2004) found that nifedipine decreases the CRP levels in the coronary sinus of patients with angina pectoris, after long-term treatment.

A further beneficial aspect of the use of statins or certain dihydropyridine calcium-channel blockers relies on their capability of protecting the myocardium from the damages associated to cardiovascular events such as myocardial infarction or stroke.

For example, it has been found that simvastatin preserves the ischemic-reperfused myocardium in normocholesterolemic rat hearts (Allan M et al Circulation 1999, 100, 178-184), whereas Sakaguchi et al (Circulation 2004, 68, 241-246) demonstrated that the CCBs manidipine and benidipine display a relevant cardioprotection in an in vitro model of ischemia-reperfusion (isolated rat hearts).

In view of what reported above, it would be highly advantageous to provide an effective combination of an antihypertensive agent and a statin able of acting in an additive or synergistic way on the risk factors of a cardiovascular diseases, further provided with a cardioprotective effect.

### PRIOR ART

In Imai Y et al (Clin Exper Hyper 1999, 21(8), 1345-1355) the effect of pravastatin on total cholesterol level in patients with hypertension in addition to mild renal dysfunction and hyperlipidemia was investigated.

Among other parameters, blood pressure, serum total cholesterol, HDL cholesterol and triglycerides were measured.

The subjects were treated with dihydropyridine CCBs through the study and, according to Table 2, six of them received 16.0 ± 5.5 mg of manidipine.

The groups of patients receiving the various dihydropyridines were not identified and the obtained results have been reported altogether, independently from the type of treatment.

The aim of the study was to investigate if pravastatin is able to decrease the serum total- and LDL-cholesterol levels in patients with mild renal dysfunction. The results demonstrated that pravastatin treatment reduced serum total cholesterol level from 251.4 mg/dl to 218.2 mg/dl, and hence in a lesser extent (about 13%) than that reported in the prior art for pravastatin alone (more than 20%).

The blood pressure did not change through the study.

The paper from Jukema et al issued on Arterioscler Thromb Vasc Biol. 1996 16(3), 425-30 deals with a retrospective analysis of a clinical investigation called the Regression Growth Evaluation Statin Study (REGRESS) in order to evaluate the effects of pravastatin on the progression of atherosclerosis in patients treated with CCBs.

However, the REGRESS trial was not designed to study the effect of CCB administration, and the authors themselves recognized that no definite conclusions can be drawn concerning the beneficial effect of adding a CCB to lipid-lowering therapy (page 429, col.2, lines 11-14). Moreover, the authors also remarked that whether all CCBs or only some of these drugs are capable of extending the antiatherosclerotic effect of pravastatin was not known.

Manidipine is not mentioned among the CCBs.

In view of the possible beneficial effects on atherosclerosis, combinations of a dihydropyridine calcium channel blocker with a lipid lowering agent, in particular a statin, have also been disclosed in the patent literature.

WO 99/11259 ('259) in the name of Pfizer discloses pharmaceutical combinations of amlodipine and atorvastatin and pharmaceutically acceptable salts thereof, kits containing such combinations and methods of using such combinations to treat subjects suffering from angina pectoris, atherosclerosis, combined hypertension and hyperlipidemia and to treat subjects presenting symptoms of cardiac risk.

In the text it is reported that the antiatherosclerotic, antianginal, antihypertensive and hypolipidemic effects of the combination are greater than the anti-anginal effects achieved by administering the two active ingredients separately. It is also reported that the effect of the combination is for managing cardiac risk in a mammal at risk of suffering an adverse cardiac event, which effect is greater than the sum of the cardiac risk management effects achieved by administering the two active ingredients separately.

In the Examples, the utility of the combination of the invention is demonstrated by the activity in clinical protocols referred to the treatment of atherosclerosis, angina, hypertension and to the management of cardiac risk.

In particular, the combination is found to be effective in slowing or arresting the progression or causing regression of existing coronary artery disease.

WO 99/11260 ('260) and WO 99/11263 ('263) are similar applications relating to pharmaceutical combinations of atorvastatin or a pharmaceutically acceptable salt thereof, and an antihypertensive agents which is not amlodipine and salts thereof and to pharmaceutical combinations of amlodipine or a pharmaceutically acceptable acid addition salt thereof, and statins or pharmaceutically acceptable salts thereof.

Also WO 00/64443, in the name of Mason, refers to a combination of amlodipine with either atorvastatin or an atorvastatin metabolite. The combination shows a synergistic antioxidant effect on lipid peroxidation in human low-density lipoproteins (LDL) and membrane vesicles enriched with polyunsaturated fatty acids.

More recently Fogari et al (J Hypertens 22 (Suppl 2) 2004, S365) have evaluated the effect of amlodipine-atorvastatin combination on plasma tissue plasminogen activator (t-PA) and inhibitor (PAI-1) activity in hypertensive, hypercholesterolemic patients with insulin resistance which are characterized by impaired fibrinolysis.

The results suggest that in hypertensive, hypercholesterolemic patients with impaired insulin sensitivity amlodipine-atorvastatin combination improves the fibrinolytic balance and decreases blood pressure more than single monotherapy.

WO 01/21158 in the name of Bayer relates to a combination of at least one dihydropyridine compound such as nifedipine or lacidipine, preferably nifedipine and an HMG-CoA reductase inhibitor, preferably cerivastatin, to the use of this combination for treating cardiovascular diseases, to medicaments containing this combination and to the production of the same.

The inventors generically state that the combination of the invention shows a synergistic antioxidant effect so the doses of the two active ingredients can be reduced.

They also state that the combination of nifedipine and cerivastatin turned out to be effective for the treatment of hypertension, heart failure and other cardiovascular diseases. Only examples of formulations are reported.

Further combinations proposed in the literature between a calcium-channel blocker and a statin comprise amlodipine and simvastatin, lacidipine and simvastatin, amolodipine and lovastatin, nifedipine and lovastatin and felodipine and simvastatin.

WO 01/15744 refers to a method for treating a subject to reduce the risk of a cardiovascular disorder, comprising selecting and administering to a subject who is known to have an above-normal level of markers of systemic inflammation, an agent selected from a calcium channel blocker, a β-adrenergic receptor blocker, a cyclooxygenase-2 inhibitor, or an angiotensin system inhibitor.

In the test it is generically stated that the method can further comprise co-administering other agents including a lipid reducing agent.

Among the markers of systemic inflammation, the CRP and the soluble cellular adhesion molecule ICAM-1 are mentioned.

Although several compositions comprising an antihypertensive agent, and in particular a dihydropyridine, in combination with a lipid reducing agent, and in particular a statin, have been proposed in the prior art there is still a need for a more efficacious medicament for the prevention and treatment of cardiovascular diseases.

### OBJECT OF THE INVENTION

The present invention provides a medicament comprising (±)-1,4-dihydro-2,6-dimethyl-4-(3-nitrophenyl)-3,5-pyridinedicarboxylic acid 2-[4-(diphenylmethyl)-1-piperazinyl]ethyl methyl ester (manidipine) or a physiologically acceptable salt thereof and simvastatin or a physiologically acceptable salt thereof.

### LEGEND TO THE FIGURE

The Figure shows the time course of left ventricular end-diastolic pressure (LVEDP) in perfused rat heart preparations submitted to ischemia-reperfusion. The hearts were excised from rats orally treated for 5 consecutive days with manidipine and simvastatin alone or in combination. Bar graph shows the area under curve (AUC) quantification related to LVEDP curves *(right panel).* Data are means ± SEM of 8 hearts per group. **P* < 0.05, ***P* < 0.01, ****P* < 0.001 vs control; ^{#}*P* < 0.01 vs simvastatin alone.

### DETAILED DESCRIPTION OF THE INVENTION

The invention relates to (±)-1,4-dihydro-2,6-dimethyl-4-(3-nitrophenyl)-3,5-pyridinedicarboxylic acid 2-[4-(diphenylmethyl)-1-piperazinyl]ethyl methyl ester (manidipine) or a physiologically acceptable salt thereof in combination with simvastatin or a physiologically acceptable salt thereof to protect the myocardium from the damages associated to post-ischemic ventricular dysfunction at reperfusion

In a particular embodiment, the invention relates to a medicament comprising a fixed combination of manidipine or a physiologically acceptable salt thereof with simvastatin or a physiologically acceptable salt thereof.

In a further embodiment, the invention also relates to a kit comprising:
a) a therapeutically effective amount of manidipine or a physiologically acceptable salt thereof and a pharmaceutically acceptable carrier or diluent in a first unit dosage form; b) a therapeutically effective amount of simvastatin or a physiologically acceptable salt thereof and a pharmaceutically acceptable carrier or diluent in a second unit dosage form; and c) container means for containing said first and second dosage forms.

It has indeed been found, in a preliminary clinical trial, that manidipine in the combinations of the present invention acts in an additive and/or synergistic way with the statin on multiple risk factors of atherosclerosis and inflammation.

The combined administration of manidipine with simvastatin significantly reduces both systolic and diastolic blood pressure (SBP and DBP) in comparison to manidipine alone and decreases the total cholesterol levels.

The combination also improves the fibrinolytic balance and this represents a further advantage since impaired fibrinolysis as well has been described as a risk factor of cardiovascular diseases.

Moreover the combined treatment gives rise to a synergistic effect on the reduction of the ICAM-1 level in comparison to each single medicament.

An additive effect is also produced on the reduction of CRP levels, a further marker associated to inflammation and to the risk of developing a cardiovascular disease.

The reduction of the markers of inflammation is particularly surprising and never disclosed before for a combination between a dihydropyridine calcium channel blocker with a statin.

Cardioprotective effects of the combination of the invention of manidipine with simvastatin were also investigated.

In a study carried out utilizing isolated perfused rat heart, it has been found that the combined administration of manidipine with simvastatin, given orally for 5 consecutive days, causes a marked inhibition of post-ischemic ventricular dysfunction. The reduction of ventricular stiffness and recovery of myocardial contractility elicited by said combination appear to be consistent with a pharmacodynamic interaction of synergistic type.

By virtue of the findings disclosed in the present application, manidipine in combination with simvastatin would turn out to be useful for both efficaciously treating or preventing cardiovascular diseases and for protecting patients from myocardium damages associated to cardiovascular events such as myocardial infarction and stroke.

Manidipine or a physiologically acceptable salt thereof and the statin or a physiologically acceptable salt thereof can be administered separately or together, preferably together in a fixed amount and quantitative ratio as a fixed combination.

Manidipine is preferably used in the form of hydrochloride salt.

The dosage amount of manidipine in the fixed combination is comprised between 5 and 30 mg. Preferably, the dosage amount of manidipine as hydrochloride salt is between 10 and 20 mg.

The ratios in which manidipine and the statin may be used in the fixed combination of the invention are variable.

Advantageously, the statin is administered in the following dosage amounts:
simvastatin, generally 2.5 mg to 160 mg and preferably 10 mg to 80 mg;

In one of the preferred fixed combinations of the invention, manidipine hydrochloride in a dosage amount of 10 to 20 mg is combined with simvastatin in a dosage amount of 10 to 80 mg, preferably 40 to 80 mg.

The combinations of the present invention are advantageously administered in the form of a pharmaceutical composition for oral administration comprising a pharmaceutically acceptable carrier or diluent.

The pharmaceutical compositions for oral administration can advantageously be in the form of tablets or capsules, preferably tablets.

Otherwise, when mandipine and the statin are administered separately, the individual components can be formulated separately. In this case, the two individual components do not unconditionally have to be taken at the same time, but in some cases taking at different times can be advantageous to achieve optimal effects. In particular manidipine shall be taken in the morning and the statin in the evening. In the case of such a separate administration, the formulation of the two individual components, for example tablets or capsule, can be packed at the same time in a suitable container mean. Such separate packaging of the components in a suitable container mean is also described as a kit.

Therefore, this invention is also directed to a kit, comprising: a) a therapeutically effective amount of manidipine or a physiologically acceptable salt thereof and a pharmaceutically acceptable carrier or diluent in a first unit dosage form; b) a therapeutically effective amount of simvastatin or a physiologically acceptable salt thereof and a pharmaceutically acceptable carrier or diluent in a second unit dosage form; and c) container means for containing said first and second dosage forms.

The combination of the invention has a broad and varied spectrum of action. It is mainly employed for the treatment or prevention of a cardiovascular disease such as angina pectoris, atherosclerosis, combined hypertension and hyperlipidemia, coronary heart disease or coronary artery disease, myocardial infarction and stroke. However it may also be useful in the treatment of other cardiovascular diseases including restenosis, transplant arteriopathy, transitory ischemic attacks, vascular thrombosis, hearth failure and cardiac arrest.

The following examples illustrate the invention in greater detail.

### Example 1 - Effect of manidipine and simvastatin, alone and in combination, in hypertensive, hypercholesterolemic patients

The aim of this study was to evaluate the effect of the manidipine-simvastatin combination on plasma tissue plasminogen activator (t-PA) and inhibitor (PAI-1) activity, on plasma intercellular adhesion molecule type-1 (ICAM-1) and on serum C-reactive protein (CRP) in hypertensive, hypercholesterolemic patients.

After 4 week placebo run-in period, 30 hypertensive hypercholesterolemic patients were randomized to a manidipine 20 mg or simvastatin 40 mg or a manidipine-simvastatin fixed combination treatment, according to 3 x 3 cross-over design; each treatment had 12 week duration.

The last day of the placebo run-in period and of each treatment period blood pressure was measured and a venous blood sample was taken (at the same hour in the morning) to evaluate plasma t-PA and PAI-1 activity, TC plasma, ICAM-1 and CRP.

The main results are shown in the following Table 1.

**Table 1**

| | Placebo | Manidipine | Simvastatin | Combination |
|---|---|---|---|---|
| SBP (mmHg) | 159.8±12.1 | 140.5±10.7 ** | 155.4+11.9 * | 137.1±9.3 **** |
| DBP (mmHg) | 100.7±6.2 | 84.1±4.8 ** | 98.2±6.2 | 81.9±4.7 *** |
| t-PA (U/ml) | 0.49±0.13 | 0.71±0.19 * | 0.51±0.14 | 0.73±0.20 * |
| PAI-1 (U/ml) | 24.6±12.3 | 24.9±12.6 | 16.1±7.3 * | 15.9±7.1 * |
| TC (mg/dl) | 254.9±28.2 | 253.8±29.6 | 202.2±26.4 * | 201.4±26.5 ** |
| ICAM-1 (ng/ml) | 237.8±35.6 | 220.2±29.8 * | 207.4±26 * | 190.1±24.6 ** |
| CRP (mg/dl) | 0.39±0.22 | 0.34±0.20 | 0.16±0.12 * | 0.14±0.08 * |

| | | | | |
|---|---|---|---|---|
| *p< 0.05; **p< 0.01; ***p<0.001 vs placebo | | | | |

As it can be appreciated from the reported results, manidipine monotherapy was significantly effective in reducing both systolic and diastolic blood pressure (SBP and DBP) mean values. Treatment with simvastatin reduced SBP, but to a lesser extent compared with manidipine.

Combination therapy with manidipine plus simvastatin produced a significantly greater reduction in both SBP (-22.7 mm Hg, p < 0.0001 vs placebo) and DBP mean values (-18.8 mm Hg, p < 0.001 vs placebo) than either drug alone.

Simvastatin significantly reduced total cholesterol (-52.7 mg/dl, p < 0.05 vs placebo) mean value. Adding manidipine did not significantly modify the lipid-lowering effect of simvastatin.

Treatment with manidipine alone did not affect plasma PAI-1 activity, whereas significantly increased the t-PA activity (+0.22 U/ml, p < 0.05 vs placebo). Simvastatin monotherapy significantly decreased PAI-1 activity (- 8.5 U/ml, p < 0.05 vs placebo) and did not affect significantly t-PA activity.

The combination produced significantly greater reduction in PAI-1 activity (-8.7 U/ml, p < 0.05 vs placebo) and increase in t-PA activity (+0.24 U/ml, p < 0.05 vs placebo), indicating that significantly improved the fibrinolytic balance.

Both manidipine (-17.6 ng/ml, p < 0.05 vs placebo) and simvastatin (-30.4 ng/ml, p < 0.05 vs placebo) were effective in reducing the ICAM-1 levels. Interestingly, the combination therapy was found to further reduce the ICAM-1 mean value (-47.7 ng/ml, p < 0.01 vs placebo).

An additive effect seems also to be produced by the combined treatment on the reduction of CRP levels, the other marker that is associated to a relative risk of developing a cardiovascular disease.

The results achieved, in particular in view of the effects on the ICAM-1 and CRP suggest that the combination of the present invention could represent an useful treatment for lowering the risk factors of cardiovascular diseases.

### Example 2 - Effect of manidipine and simvastatin, alone and in combination in preventing the myocardium damages induced by ischemia-reperfusion

The aim of this study was to evaluate the protective effects of the manidipine-simvastatin combination against myocardial ischemia-reperfusion damage in the rat.

The study was performed according to a method described previously (Rossoni G et al Pharmacol Exp Ther. 2003 307(2), 633-9).

Male Wistar rats were randomly divided into six groups (n = 6-8 rats per group). Manidipine (1 mg/kg and 3 mg/kg) simvastatin (1 mg/kg) were administered alone or in combination by oral gavage once a day for five consecutive days.

The following parameters were determined:
i) the left ventricular end-diastolic pressure (LVEDP) and the left ventricular developed pressure (LVDevP; peak left ventricular systolic pressure minus LVEDP);
ii) the activities of creatine kinase (CK) and lactate dehydrogenase (LDH) in the heart perfusate.

The data were expressed as mean ± S.E.M (standard error of the mean).

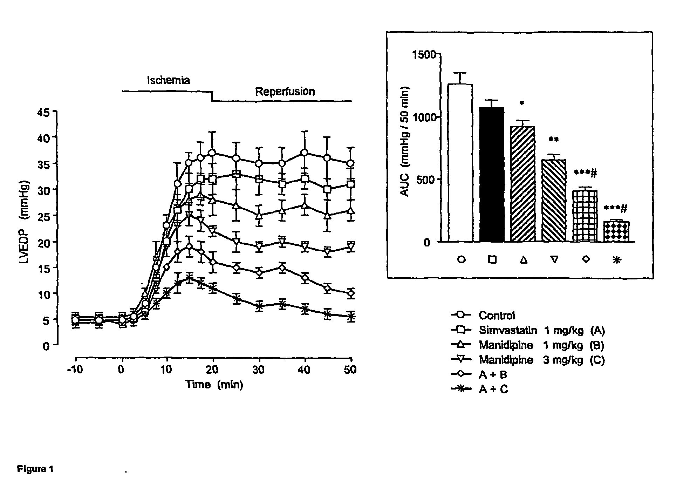

In heart preparations from control animals, ischemia induced after a standstill a progressive rise in LVEDP, peaking in approximately 20 min (from 5 ± 1 to 37 ± 4 mmHg; P < 0.001), which remained substantially unchanged even during reperfusion. In heart preparations from rat orally treated with manidipine, the rise in LVEDP during ischemia and reperfusion periods was significantly reduced according to the dose used. On the contrary, in heart preparations obtained from simvastatin (1 mg/kg)-treated rats, the rise of LVEDP values was almost similar to that of vehicle-treated rats. When hearts obtained from rats treated with combined treatments (manidipine 1 or 3 mg/kg + simvastatin 1 mg/kg) were subjected to ischemia-reperfusion, the values of LVEDP were significantly lower than those of single treatments (P < 0.01), leading at the end of reperfusion to a relevant reduction in left ventricular stiffness (Figure 1). The results clearly show that the statin administered at a dose *per se* almost ineffective (1 mg/kg), when combined with manidipine (1 or 3 mg/kg) provides a remarkable anti-ischemic effect, significantly greater than that observed with manidipine alone. The beneficial effects obtained with manidipine/simvastatin combination were confirmed by greater recovery of contractility and reduction of CK and LDH activities in heart perfusates during the reperfusion period. Taken together, these results appear to indicate a synergistic interaction between manidipine and simvastatin in reducing ventricular contracture during ischemia and ventricular dysfunction at reperfusion.

The combination of manidipine with a statin can therefore represent an efficacious therapy for protecting the myocardium from the damages associated to post-ischemic ventricular dysfunction.

## Claims

1. A pharmaceutical composition suitable for oral administration comprising:
(a) (±)-1,4-dihydro-2,6-dimethyl-4-(3-nitrophenyl)-3,5-pyridinedicarboxylic acid 2-[4-(diphenylmethyl)-1-piperazinyl]ethyl methyl ester (manidipine) or a physiologically acceptable salt thereof;
(b) simvastatin or a physiologically acceptable salt thereof; and
(c) a pharmaceutically acceptable carrier or diluent;
wherein manidipine and simvastatin are present in a fixed amount and quantitative ratio as fixed combination.

2. The pharmaceutical composition according to claim 1, wherein manidipine is in the form of hydrochloride salt.

3. The pharmaceutical composition according to claim 2, wherein manidipine is present in an amount comprised between 5 and 30 mg.

4. The pharmaceutical composition according to claim 3, wherein manidipine is present in an amount comprised between 10 and 20 mg.

5. The pharmaceutical composition according to any one of claims 2 to 4, wherein simvastatin is present in an amount comprised between 2.5 and 160 mg.

6. The pharmaceutical composition according to claim 5, wherein simvastatin is present in an amount comprised between 40 and 80 mg.

7. The pharmaceutical composition according to any one of claims 1 to 6, wherein said pharmaceutical composition is in form of a tablet or a capsule.

8. A kit, comprising: a) a therapeutically effective amount of manidipine or a physiologically acceptable salt thereof and a pharmaceutically acceptable carrier or diluent in a first unit dosage form; b) a therapeutically effective amount of simvastatin or a physiologically acceptable salt thereof and a pharmaceutically acceptable carrier or diluent in a second unit dosage form; and c) a container mean for containing said first and second dosage forms.

9. The kit according to claim 8, wherein the amount of manidipine is comprised between 5 mg and 30 mg.

10. The kit according to claim 8 or 9, wherein the amount of simvastatin is comprised between 2.5 mg and 160 mg.

11. The compound manidipine or a physiologically acceptable salt in combination with simvastatin or a physiologically acceptable salt for use to protect the myocardium from the damages associated to post-ischemic ventricular dysfunction at reperfusion.

## Patentansprüche

1. Pharmazeutische Zusammensetzung, geeignet zur oralen Verabreichung, umfassend:
(a) (d-)-1,4-Dihydro-2,6-dimethyl-4-(3-nitrophenyl)-3,5-pyridindicarbonsäure-2-[4-(diphenylmethyl)-1-piperazinyl]ethylmethylester (Manidipin) oder ein physiologisch verträgliches Salz davon;
(b) Simvastatin oder ein physiologisch verträgliches Salz davon; und
(c) einen pharmazeutisch verträglichen Träger oder ein pharmazeutisch verträgliches Verdünnungsmittel;
wobei Manidipin und Simvastatin in einer festen Menge und quantitativem Verhältnis als feste Kombination vorhanden sind.

2. Pharmazeutische Zusammensetzung gemäß Anspruch 1, wobei Manidipin in der Form des Hydrochloridsalzes vorhanden ist.

3. Pharmazeutische Zusammensetzung gemäß Anspruch 2, wobei Manidipin in einer Menge, umfasst von zwischen 5 und 30 mg, vorhanden ist.

4. Pharmazeutische Zusammensetzung gemäß Anspruch 3, wobei Manidipin in einer Menge, umfasst von zwischen 10 und 20 mg, vorhanden ist.

5. Pharmazeutische Zusammensetzung gemäß einem beliebigen der Ansprüche 2 bis 4, wobei Simvastatin in einer Menge, umfasst von zwischen 2,5 und 160 mg, vorhanden ist.

6. Pharmazeutische Zusammensetzung gemäß Anspruch 5, wobei Simvastatin in einer Menge, umfasst von zwischen 40 und 80 mg, vorhanden ist.

7. Pharmazeutische Zusammensetzung gemäß einem beliebigen der Ansprüche 1 bis 6, wobei die pharmazeutische Zusammensetzung in Form einer Tablette oder einer Kapsel vorliegt.

8. Kit, umfassend: a) eine therapeutisch wirksame Menge von Manidipin oder einem physiologisch verträglichen Salz davon und einen pharmazeutisch verträglichen Träger oder ein pharmazeutisch verträgliches Verdünnungsmittel in einer ersten Einheitsdosierungsform; b) eine therapeutisch wirksame Menge von Simvastatin oder einem physiologisch verträglichen Salz davon und einen pharmazeutisch verträglichen Träger oder ein pharmazeutisch verträgliches Verdünnungsmittel in einer zweiten Einheitsdosierungsform; und c) einen Behälter zur Aufnahme der ersten und zweiten Dosierungsform.

9. Kit gemäß Anspruch 8, wobei die Menge von Manidipin umfasst ist von zwischen 5 mg und 30 mg.

10. Kit gemäß Anspruch 8 oder 9, wobei die Menge an Simvastatin umfasst ist von zwischen 2,5 mg und 160 mg.

11. Die Verbindung Manidipin oder ein physiologisch verträgliches Salz in Kombination mit Simvastatin oder einem physiologisch verträglichen Salz zur Verwendung, um das Myocardium vor den mit post-ischämischer ventrikulärer Dysfunktion bei Reperfusion assoziierten Schäden zu schützen.

## Revendications

1. Composition pharmaceutique appropriée pour l'administration orale comprenant :
a) 2-[4-(diphénylméthyl)-1-pipérazinyl]éthyl méthyl ester de l'acide (±)-1,4-dihydro-2,6-diméthyl-4-(3-nitrophényl)-3,5-pyridinedicarboxylique (manidipine) ou un sel physiologiquement acceptable de celui-ci ;
b) simvastatine ou un sel physiologiquement acceptable de celle-ci ; et
c) un support ou diluant pharmaceutiquement acceptable ;
dans laquelle la manidipine et la simvastatine sont présentes dans une quantité fixe et un rapport quantitatif en tant que combinaison fixe.

2. Composition pharmaceutique selon la revendication 1, dans laquelle la manidipine est sous la forme d'un sel hydrochloride.

3. Composition pharmaceutique selon la revendication 2, dans laquelle la manidipine est présente dans une quantité comprise entre 5 et 30 mg.

4. Composition pharmaceutique selon la revendication 3, dans laquelle la manidipine est présente dans une quantité comprise entre 10 et 20 mg.

5. Composition pharmaceutique selon l'une quelconque des revendications 2-4, dans laquelle la simvastatine est présente dans une quantité comprise entre 2,5 et 160 mg.

6. Composition pharmaceutique selon la revendication 5, dans laquelle la simvastatine est présente dans une quantité comprise entre 40 et 80 mg.

7. Composition pharmaceutique selon l'une quelconque des revendications 1 à 6, ladite composition pharmaceutique étant sous forme d'un comprimé ou d'une capsule.

8. Kit comprenant :
a) une quantité thérapeutiquement efficace de manidipine ou d'un sel physiologiquement acceptable de celle-ci et un support ou diluant pharmaceutiquement acceptable sous forme d'une première dose unitaire;
b) une quantité thérapeutiquement efficace de simvastatine ou d'un sel physiologiquement acceptable de celle-ci et un support ou diluant pharmaceutiquement acceptable sous forme d'une deuxième dose unitaire; et
c) un récipient en tant que moyen pour contenir lesdites première et deuxième formes de dose.

9. Kit selon la revendication 8, dans lequel la quantité de manidipine est comprise entre 5 mg et 30 mg.

10. Kit selon la revendication 8 ou 9, dans lequel la quantité de simvastatine est comprise entre 2,5 mg et 160 mg.

11. Le composé manidipine ou un sel physiologiquement acceptable en combinaison avec la simvastatine ou un sel pysiologiquement acceptable pour une utilisation dans la protection du myocarde contre les dommages associés au dysfonctionnement ventriculaire post-ischémique lors de la réperfusion.
